(19) Europäisches Patentamt
European Patent Office
Office européen des brevets

(11) EP 0 754 681 B1

(12) FASCICULE DE BREVET EUROPEEN

(45) Date de publication et mention de la délivrance du brevet:
**04.03.1998 Bulletin 1998/10**

(51) Int. Cl.[6]: **C07D 209/08**, A61K 7/13

(21) Numéro de dépôt: **96401413.8**

(22) Date de dépôt: **26.06.1996**

# (54) Dérivés N-substitués du 4-hydroxyindole et compositions de teinture des fibres kératiniques les contenant

N-substituierte 4-Hydroxyindolderivate und Zusammensetzungen zur Färbung keratinischer Fasern die sie enthalten

N-substituted 4-hydroxyindole derivatives and compositions for dyeing keratinous fibers containing them

(84) Etats contractants désignés:
**AT BE CH DE ES FR GB IT LI NL SE**

(30) Priorité: **13.07.1995 FR 9508566**

(43) Date de publication de la demande:
**22.01.1997 Bulletin 1997/04**

(73) Titulaire: **L'OREAL**
**75008 Paris (FR)**

(72) Inventeurs:
- **Terranova, Eric**
  **92600 Asnieres (FR)**
- **Fadli, Aziz**
  **93150 Le Blanc Mesnil (FR)**
- **Lagrange, Alain**
  **77450 Coupvray (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 428 441**
**WO-A-92/17157**
**JP-A- 2 059 555**
**US-A- 3 503 990**
- **Helv. Chim. Acta (1959), Vol.42, 2073-2102**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.15.12/3.4

## Description

L'invention a pour principal objet une composition pour la teinture d'oxydation des fibres kératiniques et en particulier des fibres kératiniques humaines telles que les cheveux contenant, à titre de coupleur, au moins un dérivé N-substitué du 4-hydroxy indole, et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet européen EP-A-428 441, des compositions pour la teinture d'oxydation des fibres kératiniques renfermant, à titre de coupleurs, au moins un dérivé de 4-hydroxy indole pouvant être N-substitué par un radical alkyle en $C_1$-$C_4$. De telles compositions permettent d'atteindre des gammes variées de nuances mais elles ne sont cependant pas entièrement satisfaisantes notamment du point de vue de la tenue des colorations obtenues vis à vis des diverses agressions que peuvent subir les cheveux et en particulier vis à vis de la lumière.

Or, la demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des dérivés particuliers du 4-hydroxyindole, à savoir des dérivés convenablement N-substitués. Ces composés (pour partie nouveaux en soi) sont de plus aisément synthétisables.

Cette découverte est à la base de la présente invention.

L'invention a donc pour objet une composition pour la teinture des fibres kératiniques et en particulier des fibres kératiniques humaines telles que les cheveux, caractérisée par le fait qu'elle comprend, dans un milieu approprié pour la teinture :

- à titre de coupleur, au moins un dérivé N-substitué du 4-hydroxy indole de formule (I), et/ou au moins l'un de ses sels d'addition avec un acide :

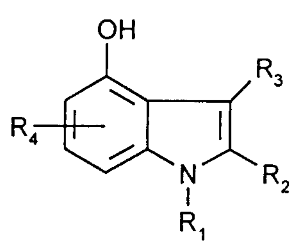

(I)

dans laquelle :

- $R_1$ représente un radical monohydroxyalkyle en $C_1$-$C_4$ ; polyhydroxyalkyle en $C_2$-$C_4$ ; alcoxy($C_1$-$C_4$)alkyle en $C_1$-$C_4$ ; hydroxyalcoxy($C_1$-$C_4$)alkyle en $C_1$-$C_4$ ; aminoalkyle en $C_1$-$C_4$ ; aminoalkyle en $C_1$-$C_4$ dont l'amine est mono ou disubstituée par un groupement alkyle en $C_1$-$C_4$, par un groupement acétyle, par un groupement monohydroxyalkyle en $C_1$-$C_4$ ou par un groupement polyhydroxyalkyle en $C_2$-$C_4$ ; alkyle($C_1$-$C_4$)thioalkyle en $C_1$-$C_4$, monohy-

droxyalkyl($C_1$-$C_4$)thioalkyle en $C_1$-$C_4$ ; polyhydroxyalkyl($C_2$-$C_4$)thioalkyle en $C_1$-$C_4$ ; carboxyalkyle en $C_1$-$C_4$ ; alcoxy($C_1$-$C_4$)carbonylalkyle en $C_1$-$C_4$ ; cyanoalkyle en $C_1$-$C_4$ ; trifluoroalkyle en $C_1$-$C_4$ ; halogénoalkyle en $C_1$-$C_4$ ; phosphoalkyle en $C_1$-$C_4$ ou sulfoalkyle en $C_1$-$C_4$ ;

- $R_2$ et $R_3$, identiques ou différents, représentent un atome d'hydrogène ou d'halogéne ; un radical alkyle en $C_1$-$C_4$ ; carboxyle ; alcoxy($C_1$-$C_4$)carbonyle ou formyle ;
- $R_4$ représente un atome d'hydrogène ou d'halogéne ; un radical alkyle en $C_1$-$C_4$ ; alcoxy en $C_1$-$C_4$ ; acétylamino ; monohydroxyalkyle en $C_1$-$C_5$ ; polyhydroxyalkyle en $C_2$-$C_4$ ; alcoxy($C_1$-$C_4$)alkyle en $C_1$-$C_4$ ; thiophène ; furane ; phényle ; phényle substitué par un atome d'halogéne, un alkyle en $C_1$-$C_4$, un trifluorométhyle, un alcoxy en $C_1$-$C_4$, un amino ou par un amino mono- ou disubstitué par un radical alkyle en $C_1$-$C_4$ ; alkyl($C_1$-$C_4$)aminoalkyle en $C_1$-$C_4$ ou dialkyl($C_1$-$C_4$)aminoalkyle en $C_1$-$C_4$ ;
- et au moins une base d'oxydation.

Dans la formule (I) ci-dessus, les groupements alkyle et alcoxy peuvent être linéaires ou ramifiés et parmi les atomes d'halogène, on peut citer le chlore, le brome, l'iode et le fluor.

Les coupleurs de formule (I) conforme à l'invention se démarquent des produits connus du documents EP-A-428 441 précité, essentiellement par la nature du substituant $R_1$ en position 1-N.

Les colorations obtenues avec la composition de teinture conforme à l'invention, sont de nuances variées, puissantes, peu sélectives et présentent d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables vis à vis de la lumière.

Parmi les dérivés N-substitués du 4-hydroxyindole de formule (I), utilisables à titre de coupleurs dans les compositions conformes à l'invention, on peut notamment citer :

- le 4-hydroxy 1-N-(β-hydroxyéthyl) indole,
- le 4-hydroxy 1-N-(β-hydroxypropyl) indole,
- le 1-N-(β,γ-dihydroxypropyl) 4-hydroxy indole,
- le 4-hydroxy 1-N-(β-hydroxyéthyl) 5-méthyl indole,
- le 4-hydroxy 1-N-(β-hydroxypropyl) 5-méthyl indole,
- le 1-N-(β,γ-dihydroxypropyl) 4-hydroxy 5-méthyl indole,
- le 4-hydroxy 1-N-(β-hydroxyéthyl) 6-méthyl indole,
- le 4-hydroxy 1-N-(β-hydroxypropyl) 6-méthyl indole,
- le 1-N-(β,γ-dihydroxypropyl) 4-hydroxy 6-méthyl indole,
- le 5-benzyl 4-hydroxy 1-N-(β-hydroxyéthyl) indole,
- le 5-benzyl 4-hydroxy 1-N-(β-hydroxypropyl) indole,
- le 5-benzyl 1-N-(β,γ-dihydroxypropyl) 4-hydroxy indole,
- le 4-hydroxy 1-N-(β-hydroxyéthyl) 5-β-hydroxyéthyl indole,
- le 4-hydroxy 5-β-hydroxyéthyl 1-N-(β-hydroxypropyl) indole,
- le 1-N-(β,γ-dihydroxypropyl) 4-hydroxy 5-β-hydroxyéthyl indole,
- le 4-hydroxy 1-N-(β-hydroxyéthyl) 5-β,γ-dihydroxypropyl indole,
- le 4-hydroxy 1-N-(β-hydroxypropyl) 5-β,γ-dihydroxypropyl indole,
- le 1-N-(β,γ-dihydroxypropyl) 4-hydroxy 5-β,γ-dihydroxypropyl indole,
- le 1-N-(γ-diméthylaminopropyl) 4-hydroxy indole,
- le 1-N-éthylaminoéthyl 4-hydroxy indole

et leurs sels d'addition avec un acide.

Parmi ces dérivés N-substitués du 4-hydroxy indole, on préfère plus particulièrement :

- le 4-hydroxy 1-N-(β-hydroxyéthyl) indole,
- le 4-hydroxy 1-N-(β-hydroxypropyl) indole,
- le 1-N-(β,γ-dihydroxypropyl) 4-hydroxy indole,
- le 4-hydroxy 1-N-(β-hydroxyéthyl) 5-méthyl indole,
- le 1-N-(γ-diméthylaminopropyl) 4-hydroxy indole,

et leur sels d'addition avec un acide.

Les sels d'addition avec un acide des composés de formule (I) utilisables à titre de coupleurs dans les compositions de teinture conformes à l'invention sont notamment choisis parmi les chlorhydrates, les bromhydrates, les sulfates et les tartrates.

Le ou les dérivés N-substitués du 4-hydroxy indole de formule (I) représentent de préférence de 0,0005 à 12 % en poids environ du poids total de la composition tinctoriale, et encore plus préférentiellement de 0,005 à 6 % en poids

environ de ce poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les para-aminophénols, les ortho-aminophénols, les bases hétérocycliques, et leurs sels d'addition avec un acide.

Parmi les paraphénylènediamines utilisables à titre de bases d'oxydation dans la composition tinctoriale selon l'invention, on peut notamment citer les composés répondant à la formule (II) suivante, et leurs sels d'addition avec un acide :

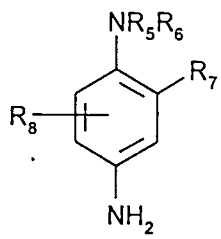

(II)

dans laquelle :

$R_5$ représente un atome d'hydrogène, un radical alkyle en $C_1$-$C_4$, monohydroxyalkyle en $C_1$-$C_4$, polyhydroxyalkyle en $C_2$-$C_4$ ou alcoxy($C_1$-$C_4$)alkyle($C_1$-$C_4$),
$R_6$ représente un atome d'hydrogène, un radical alkyle en $C_1$-$C_4$, monohydroxyalkyle en $C_1$-$C_4$ ou polyhydroxyalkyle en $C_2$-$C_4$,
$R_7$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$,
$R_8$ représente un atome d'hydrogène ou un radical alkyle en $C_1$-$C_4$.

Dans la formule (II) ci-dessus, et lorsque $R_7$ est différent d'un atome d'hydrogène, alors $R_5$ et $R_6$ représentent de préférence un atome d'hydrogène et $R_7$ est de préférence identique à $R_8$, et lorsque $R_7$ représente un atome d'halogène, alors $R_5$, $R_6$ et $R_8$ représentent de préférence un atome d'hydrogène.

Parmi les paraphénylènediamines de formule (II) ci-dessus, on peut plus particulièrement citer la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, le 4-amino 1-(β-méthoxyéthyl)amino benzène, la 2-chloro paraphénylènediamine, et leurs sels d'addition avec un acide.

Parmi les bis-phénylalkylènediamines utilisables à titre de bases d'oxydation dans la composition tinctoriale selon l'invention, on peut notamment citer les composés répondant à la formule (III) suivante, et leurs sels d'addition avec un acide :

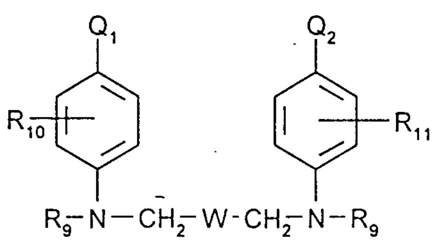

(III)

dans laquelle :

$Q_1$ et $Q_2$, identiques ou différents, représentent un radical hydroxyle ou $NHR_{12}$ dans lequel $R_{12}$ représente un atome d'hydrogène ou un radical alkyle en $C_1$-$C_4$,
$R_9$ représente un atome d'hydrogène, un radical alkyle en $C_1$-$C_4$, monohydroxyalkyle en $C_1$-$C_4$, polyhydroxyalkyle en $C_2$-$C_4$ ou aminoalkyle en $C_1$-$C_4$ dont le reste amino peut être substitué,
$R_{10}$ et $R_{11}$, identiques ou différents, représentent un atome d'hydrogène ou d'halogène ou un radical alkyle en $C_1$-

$C_4$,
W représente un radical pris dans le groupe constitué par les radicaux suivants :
$-(CH_2)_n-$ ; $-(CH_2)_m-O-(CH_2)_m-$ ; $-(CH_2)_m-CHOH-(CH_2)_m-$ et

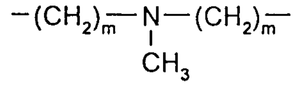 ;

dans lesquels n est un nombre entier compris entre 0 et 8 inclusivement et m est un nombre entier compris entre 0 et 4 inclusivement.

Parmi les bis-phénylalkylènediamines de formules (III) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol ou l'un de ses sels d'addition avec un acide sont particulièrement préférés.

Parmi les para-aminophénols utilisables à titre de bases d'oxydation dans la composition tinctoriale selon l'invention, on peut notamment citer les composés répondant à la formule (IV) suivante, et leurs sels d'addition avec un acide :

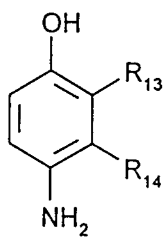

(IV)

dans laquelle :

$R_{13}$ représente un atome d'hydrogène, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$,
$R_{14}$ représente un atome d'hydrogène ou de fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),
étant entendu qu'au moins un des radicaux $R_{13}$ ou $R_{14}$ représente un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition tinctoriale selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.
Parmi dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triaminopyrimidine, et leurs sels d'addition avec un acide.
Parmi les dérivés pyrazoliques, on peut plus particulièrement citer les composés décrits dans les brevets DE 3 843 892,

DE 4 133 957 et demandes de brevet WO 94/08969 et WO 94/08970 comme le 4,5-diamino 1-méthyl pyrazole, le 3,4-diamino pyrazole, et leurs sels d'addition avec un acide.

Selon l'invention, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale selon l'invention peut également renfermer un ou plusieurs coupleurs additionnels différents des dérivés N-substitués du 4-hydroxyindole de formule (I) et/ou un ou plusieurs colorants directs de façon à faire varier ou enrichir en reflets les nuances obtenues avec les bases d'oxydation.

Les coupleurs additionnels utilisables dans la composition selon l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents ces coupleurs additionnels représentent de préférence de 0,0005 à 5 % en poids environ du poids total de la composition tinctoriale, et encore plus préférentiellement de 0,005 à 3 % en poids environ de ce poids.

Les sels d'addition avec un acide de la ou des bases d'oxydation et/ou des coupleurs additionnels utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante :

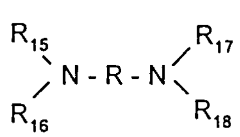

(V)

dans laquelle R est un reste propylène éventuellement substitué par un groupement hydroxyle ou un radical alkyle en $C_1$-$C_4$ ; $R_{15}$, $R_{16}$, $R_{17}$ et $R_{18}$, identiques ou différents, représentent un atome d'hydrogène, un radical alkyle en $C_1$-$C_4$ ou hydroxyalkyle en $C_1$-$C_4$.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des dérivés N-substitués du 4-hydroxyindole de formule (I) ci-dessus, à titre de coupleur, en association avec au moins une base d'oxydation pour la teinture d'oxydation des fibres kératiniques et particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Certains composés de formule (I), utilisés à titre de coupleurs dans le cadre de la présente invention, sont nouveaux et, à ce titre, constituent un autre objet de l'invention.

Ces nouveaux dérivés N-substitués du 4-hydroxy indole et leurs sels d'addition avec un acide répondent à la formule (I') suivante :

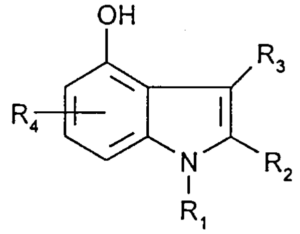

(I')

dans laquelle :

- $R_1$ représente un radical monohydroxyalkyle en $C_1$-$C_4$ ; polyhydroxyalkyle en $C_2$-$C_4$ ; alcoxy($C_1$-$C_4$)alkyle en $C_1$-$C_4$ ; hydroxyalcoxy($C_1$-$C_4$)alkyle en $C_1$-$C_4$ ; aminoalkyle en $C_1$-$C_4$ ; aminoalkyle en $C_1$-$C_4$ dont l'amine est mono ou disubstituée par un groupement alkyle en $C_1$-$C_4$, par un groupement acétyle, par un groupement monohydroxyalkyle en $C_1$-$C_4$ ou par un groupement polyhydroxyalkyle en $C_2$-$C_4$ ; alkyle($C_1$-$C_4$)thioalkyle en $C_1$-$C_4$, monohydroxyalkyl($C_1$-$C_4$)thioalkyle en $C_1$-$C_4$ ; polyhydroxyalkyl($C_2$-$C_4$)thioalkyle en $C_1$-$C_4$ ; carboxyalkyle en $C_1$-$C_4$ ; alcoxy($C_1$-$C_4$)carbonylalkyle en $C_1$-$C_4$ ; cyanoalkyle en $C_1$-$C_4$ ; trifluoroalkyle en $C_1$-$C_4$ ; halogénoalkyle en $C_1$-$C_4$ ; phosphoalkyle en $C_1$-$C_4$ ou sulfoalkyle en $C_1$-$C_4$ ;
- $R_2$ et $R_3$, identiques ou différents, représentent un atome d'hydrogène ou d'halogène ; un radical alkyle en $C_1$-$C_4$ ; carboxyle ; alcoxy($C_1$-$C_4$)carbonyle ou formyle ;
- $R_4$ représente un atome d'hydrogène ou d'halogène ; un radical alkyle en $C_1$-$C_4$ ; alcoxy en $C_1$-$C_4$ ; acétylamino ; monohydroxyalkyle en $C_1$-$C_5$ ; polyhydroxyalkyle en $C_2$-$C_4$ ; alcoxy($C_1$-$C_4$)alkyle en $C_1$-$C_4$ ; thiophène ; furane ; phényle ; phényle substitué par un atome d'halogène, un alkyle en $C_1$-$C_4$, un trifluorométhyle, un alcoxy en $C_1$-$C_4$, un amino ou par un amino mono- ou disubstitué par un radical alkyle en $C_1$-$C_4$ ; alkyl($C_1$-$C_4$)aminoalkyle en $C_1$-$C_4$ ou dialkyl($C_1$-$C_4$)aminoalkyle en $C_1$-$C_4$ ; étant entendu que :
- lorsque $R_2$ et $R_4$ représentent simultanément un atome d'hydrogène, et que $R_1$ représente un groupement méthoxyméthyle ou γ-chloro-β-hydroxypropyle, alors $R_3$ ne peut représenter un radical formyle, (correspond à deux composés connus de l'art antérieur décrits dans la demande de brevet JP 88-208937 = JP-A-2 059 555),
- et lorsque que $R_2$, $R_3$ et $R_4$ représentent simultanément un atome d'hydrogène, alors $R_1$ ne peut représenter un radical diméthylaminoéthyle, (correspond à un composé connu de l'art antérieur décrit dans Troxler et al., Helv. Chim. Acta, Vol. 42, 1959, 2073-2102).

Parmi les nouveaux dérivés N-substitués du 4-hydroxyindole de formule (I'), on peut notamment citer :

- le 4-hydroxy 1-N-(β-hydroxyéthyl) indole,
- le 4-hydroxy 1-N-(β-hydroxypropyl) indole,
- le 1-N-(β,γ-dihydroxypropyl) 4-hydroxy indole,
- le 4-hydroxy 1-N-(β-hydroxyéthyl) 5-méthyl indole,
- le 4-hydroxy 1-N-(β-hydroxypropyl) 5-méthyl indole,
- le 1-N-(β,γ-dihydroxypropyl) 4-hydroxy 5-méthyl indole,
- le 4-hydroxy 1-N-(β-hydroxyéthyl) 6-méthyl indole,
- le 4-hydroxy 1-N-(β-hydroxypropyl) 6-méthyl indole,
- le 1-N-(β,γ-dihydroxypropyl) 4-hydroxy 6-méthyl indole,
- le 5-benzyl 4-hydroxy 1-N-(β-hydroxyéthyl) indole,
- le 5-benzyl 4-hydroxy 1-N-(β-hydroxypropyl) indole,
- le 5-benzyl 1-N-(β,γ-dihydroxypropyl) 4-hydroxy indole,
- le 4-hydroxy 1-N-(β-hydroxyéthyl) 5-β-hydroxyéthyl indole,
- le 4-hydroxy 5-β-hydroxyéthyl 1-N-(β-hydroxypropyl) indole,
- le 1-N-(β,γ-dihydroxypropyl) 4-hydroxy 5-β-hydroxyéthyl indole,
- le 4-hydroxy 1-N-(β-hydroxyéthyl) 5-β,γ-dihydroxypropyl indole,
- le 4-hydroxy 1-N-(β-hydroxypropyl) 5-β,γ-dihydroxypropyl indole,
- le 1-N-(β,γ-dihydroxypropyl) 4-hydroxy 5-β,γ-dihydroxypropyl indole,
- le 1-N-(γ-diméthylaminopropyl) 4-hydroxy indole,
- le 1-N-éthylaminoéthyl 4-hydroxy indole

et leurs sels d'addition avec un acide.

Parmi ces nouveaux dérivés N-substitués du 4-hydroxy indole de formule (I'), on préfère plus particulièrement :

- le 4-hydroxy 1-N-(β-hydroxyéthyl) indole,
- le 4-hydroxy 1-N-(β-hydroxypropyl) indole,
- le 1-N-(β,γ-dihydroxypropyl) 4-hydroxy indole,
- le 4-hydroxy 1-N-(β-hydroxyéthyl) 5-méthyl indole,
- le 1-N-(γ-diméthylaminopropyl) 4-hydroxy indole,

et leurs sels d'addition avec un acide.

L'invention a également pour objet un procédé de préparation (procédé principal) des composés de formule (I'), répondant au schéma de synthèse suivant :

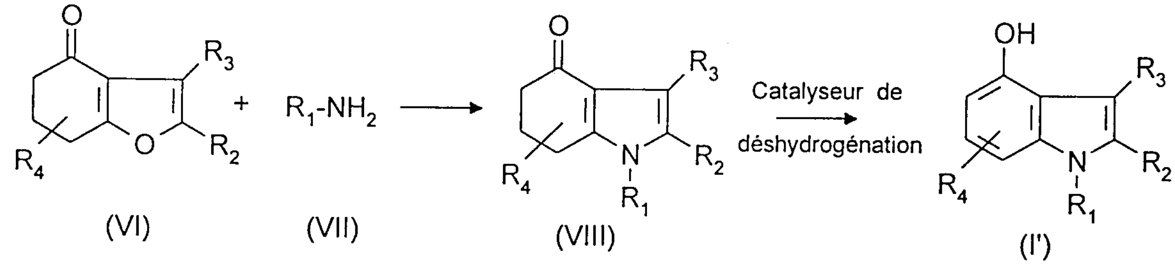


(VI) (VII) (VIII) (I')

consistant à faire réagir, dans une première étape, un 4-oxo 4,5,6,7-tetrahydro benzofurane de formule (VI), dans laquelle les radicaux $R_2$, $R_3$ et $R_4$ ont la même signification que dans la formule (I') définie ci-dessus, avec une amine substituée de formule (VII), dans laquelle le radical $R_1$ a la même signification que dans la formule (I') définie ci-dessus, dans un milieu solvant dont la température est comprise de préférence entre 80 et 160°C, pour conduire à un dérivé 4-oxo 4,5,6,7-tetrahydroindolique de formule (VIII), dans laquelle les radicaux $R_1$, $R_2$, $R_3$ et $R_4$ ont la même signification que dans la formule (I') définie ci-dessus, puis dans une deuxième étape, à aromatiser le composé de formule (VIII) par déshydrogénation catalytique en milieu solvant, à une température généralement comprise entre 150 et 220°C et de préférence comprise entre 160 et 170°C, pour conduire à un composé de formule (I') telle que définie précédemment.

Parmi les solvants utilisés lors de la première étape, on peut plus particulièrement citer les alcools inférieurs tels que l'éthanol, le n-propanol, le 1-butanol, le 2-butanol, le 2-méthyl 1-butanol, le 3-méthyl 1-butanol, le 2-méthyl 1-propanol, le n-pentanol, le 2-pentanol, le 3-méthyl 3-pentanol, le 4-méthyl 2-pentanol ou encore le 2-éthyl 1-butanol.

Selon le procédé de l'invention, on peut employer tout catalyseur de déshydrogénation approprié, par exemple un métal choisi dans le groupe constitué par le manganèse, le platine, le palladium, le rhodium, le nickel et le ruthénium, leurs oxydes, et les combinaisons de ces substances.

Les catalyseurs de déshydrogénation préférés sont le palladium ou le platine. De façon connue, le catalyseur peut être déposé sur un support inerte. Parmi ces supports inertes, on peut citer par exemple le charbon de bois neutre, le charbon neutre, l'alumine neutre, les zéolithes, les argiles, etc. On utilise de préférence du charbon neutre.

Les catalyseurs de déshydrogénation sont en général présents en une quantité comprise entre 0,2 et 5 % en poids d'équivalent métal par rapport au poids du composé de formule (VIII) à faire réagir.

Les solvants utilisés lors de la deuxième étape sont de préférence choisis parmi les solvants dont le point d'ébullition est supérieur à 150°C, tel que par exemple le diglyme dont le point d'ébullition (P.E.) est d'environ 162°C et le diisobutylcétone (P.E. d'environ 169°C).

Selon une première variante de ce procédé principal, et lorsque dans la formule (VI) le radical $R_4$ est un atome d'hydrogène, alors on peut introduire un radical $R'_4$, en position 5, différent d'un atome d'hydrogène sur les composés de formule (VIII), par réaction d'aldolisation en milieu basique. Cette première variante répond au schéma de synthèse suivant :

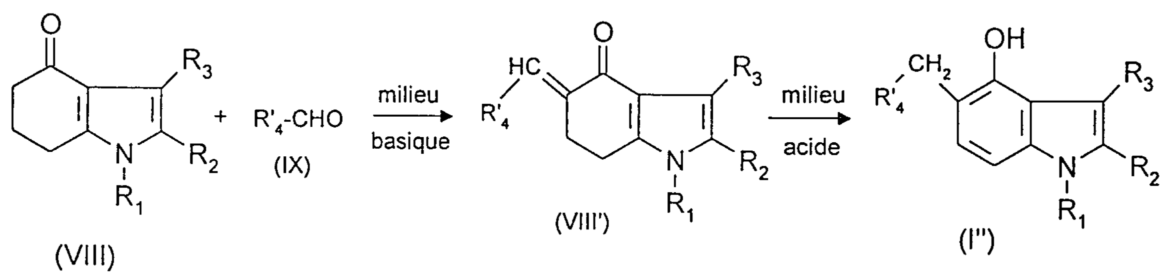


(VIII) (IX) (VIII') (I'')

consistant à faire réagir un composé de formule (VIII) et un aldéhyde de formule (IX) dans laquelle le radical $R'_4$ représente un radical alkyle en $C_1$-$C_3$, monohydroxyalkyle en $C_1$-$C_4$, polyhydroxyalkyle en $C_2$-$C_3$, alcoxyalkyle en $C_1$-$C_3$, alkyl($C_1$-$C_4$)aminoalkyle en $C_1$-$C_3$ ou dialkyl($C_1$-$C_4$)aminoalkyle en $C_1$-$C_3$, en milieu basique, pour obtenir un composé de formule (VIII') dans lesquels le radical $R'_4$ a la même signification que dans la formule (IX), qui est ensuite isomérisé en milieu acide selon les conditions classiques d'isomérisation, pour conduire à un composé de formule (I'') dans laquelle les radicaux $R_1$, $R_2$ et $R_3$ ont les mêmes significations que dans la formule (I'), et le radical $R'_4$ a la même signi-

fication que dans la formule (IX).

La réaction d'aldolisation est bien connue de l'homme du métier et est décrite par exemple dans la demande de brevet EP-A-0 377 450.

Selon une deuxième variante de ce procédé principal, on peut également fonctionnaliser la partie benzénique des composés de formule (I') obtenus à partir d'un composé de formule (VIII) dans lequel $R_4$ représente un atome d'hydrogène pour introduire un radical $R_4$ différent d'un atome d'hydrogène. Cette seconde variante répond au schéma de synthèse suivant :

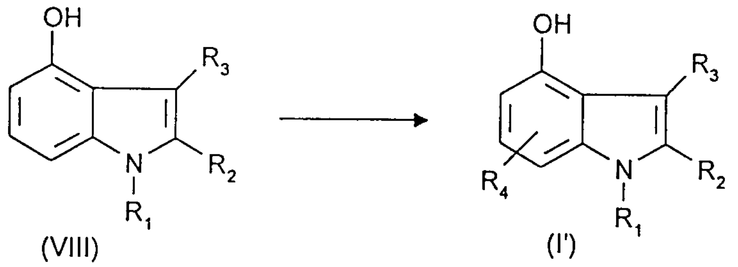

(VIII) (I')

Cette seconde variante permet notamment d'introduire :

- soit un substituant $R_4$ de type dialkyl($C_1$-$C_4$)aminométhyl, en position 5, par une réaction de MANNICH telle que décrite par exemple dans l'article de F. TROXLER & al., Helv. Chim. Acta, 51, (6), 1968 ;
- soit un substituant $R_4$ de type acétylamino par une réaction de nitration suivie d'une réaction de réduction et d'une acétylation, par les méthodes classiques bien connues de l'homme du métier ;
- soit encore un substituant $R_4$ de type halogéné, par une réaction classique d'halogénation, lorsque les radicaux $R_2$ et $R_3$ sont tous deux différents d'un atome d'hydrogène.

Cette énumération n'est bien sûr pas limitative du type de groupements $R_4$ pouvant être introduits directement sur les composés de formule (I').

Les exemples qui suivent sont destinés à illustrer l'invention sans pour autant en limiter la portée.

**EXEMPLES DE PREPARATION**

**EXEMPLE DE PREPARATION 1 : Synthèse du 4-hydroxy 1-N-(β-hydroxyéthyl) indole**

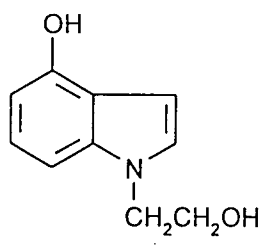

a) Préparation du 4-oxo 4,5,6,7-tétrahydro 1-N-(β-hydroxyéthyl) indole

A une solution de 136 g de 4-oxo 4,5,6,7-tétrahydro benzofurane dans 250 $cm^3$ d'éthanol, on a ajouté 80 g d'éthanolamine. La solution a été portée à 130°C pendant 6 heures. Après avoir laissé le mélange réactionnel revenir à température ambiante sous agitation, celui-ci a été coulé sur un mélange de 800 $cm^3$ d'éther isopropylique et de 200 $cm^3$

d'éther de pétrole. Un produit a cristallisé sous agitation, puis a été essoré, lavé à l'éther de pétrole et séché sous vide sur pentaoxyde de phosphore. On a recueilli 160 g du produit attendu qu'on a recristallisé dans 240 $cm^3$ d'isopropanol. On a obtenu 150 g du produit attendu dont le point de fusion était compris entre 96 et 97°C.

b) Préparation du 4-hydroxy 1-N-(β-hydroxyéthyl) indole

A une solution de 25 g de 4-oxo 4,5,6,7-tetrahydro 1-N-(β-hydroxyéthyl) indole, obtenu à l'étape précédente, dans 300 $cm^3$ de diglyme, on a ajouté 15 g de palladium sur charbon à 5 % en poids et contenant 50 % d'eau. La température du mélange a été portée et maintenue à 162°C pendant 10 heures. On a ensuite laissé revenir le mélange à une température de 40°C, puis le catalyseur a été filtré. Les solvants ont ensuite été éliminés sous vide jusqu'à obtention de 21,4 g de produit brut qui ont été repris par un mélange de 30 $cm^3$ de dichlorométhane et de 200 $cm^3$ d'éther de pétrole. Les cristaux obtenus ont été essorés, lavés à l'éther de pétrole puis séchés sous vide sur pentaoxyde de phosphore. On a obtenu 11 g de 4-hydroxy 1-N-(β-hydroxyéthyl) indole dont l'analyse élémentaire calculée pour $C_{10}H_{11}NO_2$ était :

| % | C | H | N | O |
|---|---|---|---|---|
| Calculé | 67,78 | 6,26 | 7,90 | 18,06 |
| Trouvé | 67,63 | 6,11 | 7,52 | 18,25 |

**EXEMPLE DE PREPARATION 2 : Synthèse du 4-hydroxy 1-N-(β-hydroxypropyl) indole**

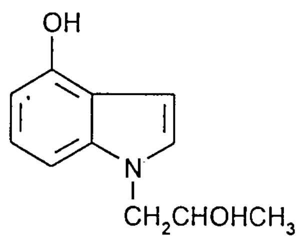

a) Préparation du 4-oxo 4,5,6,7-tétrahydro 1-N-(β-hydroxypropyl) indole

A une solution de 13,6 g de 4-oxo 4,5,6,7-tetrahydro benzofurane dans 200 $cm^3$ d'éthanol, on a ajouté 7,2 g de β-hydroxypropylamine. La solution a été portée à 150°C pendant 4 heures. L'éthanol a ensuite été évaporé sous vide. On a recueilli 18 g d'une huile qui a été utilisée telle quelle à l'étape suivante.

b) Préparation du 4-hydroxy 1-N-(β-hydroxypropyl) indole

A une solution de 19,3 g de 4-Oxo 4,5,6,7-tetrahydro 1-N-(β-hydroxypropyl) indole, obtenu à l'étape précédante, dans 300 $cm^3$ de diglyme, on a ajouté 10 g de palladium sur charbon à 5 % en poids et contenant 50 % d'eau. La température du milieu réactionnel a été portée à la température de reflux du diglyme pendant 5 heures, après élimination de l'eau par azéotropie. Le catalyseur a ensuite été filtré sur celite puis on a évaporé le diglyme. On a obtenu 17 g de produit brut. Après chromatographie sur gel de silice (Heptane/acétate d'éthyle = 1/4), on a obtenu 15 g de produit visqueux qui a cristallisé par addition de 15 $cm^3$ de dichlorométhane. Après filtration, lavage à l'éther de pétrole et séchage sous vide et sur pentaoxyde de phosphore, on a récupéré 10 g de 4-hydroxy 1-N-(β-hydroxypropyl) indole dont le point de fusion était compris entre 93 et 95 C et dont l'analyse élémentaire calculée pour $C_{11}H_{13}NO_2$ était :

| % | C | H | N | O |
|---|---|---|---|---|
| Calculée | 69,09 | 6,85 | 7,32 | 16,73 |
| Trouvée | 69,08 | 7,00 | 7,25 | 16,73 |

**<u>EXEMPLE DE PREPARATION 3</u> : Synthèse du 4-hydroxy 1-N-(β,γ-dihydroxypropyl) indole**

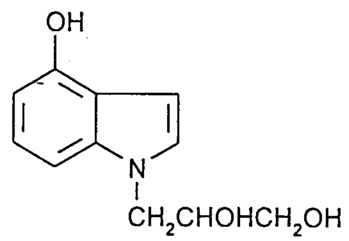

a) Préparation du 4-oxo 4,5,6,7-tétrahydro 1-N-(β,γ-dihydroxypropyl) indole

A une solution de 13,6 g de 4-oxo 4,5,6,7-tetrahydro benzofurane dans 200 $cm^3$ d'éthanol, on a ajouté 9,4 g de 2,3-dihydroxypropylamine. On a chauffé le milieu réactionnel à 150°C pendant 9 heures. Après évaporation du solvant et purification du brut sur gel de silice (Acétate d'éthyle/méthanol = 9/1), on a recueilli 11,2 g de 4-oxo 4,5,6,7-tetrahydro-1-N-(β,γ-dihydroxypropyl) indole sous forme d'une huile.

b) Préparation du 4-hydroxy 1-N-(β,γ-dihydroxypropyl)indole

A une solution de 9,4 g de 4-oxo 4,5,6,7-tetrahydro 1-N-(β,γ-dihydroxypropyl) indole obtenu à l'étape précédente, dans 50 $cm^3$ de diglyme, on a introduit 3,8 g de palladium sur charbon à 5 % en poids et contenant 50 % d'eau La température du milieu réactionnel a été portée à la température de reflux du diglyme, et l'eau a été distillée par azéotropie. Après 23 heures de réaction, le catalyseur a été filtré sur celite puis on a évaporé le diglyme sous vide. Le produit brut obtenu a été chromatographié sur gel de silice (Acétate d'éthyle/heptane = 9/1). On a obtenu 4,2 g de 4-hydroxy 1-N-(β,γ-dihydroxypropyl) indole sous forme d'une huile très visqueuse, dont l'analyse en résonance magnétique nucléaire $^1H$ et $^{13}C$ était conforme au produit attendu.

**EXEMPLES DE FORMULATION**

**<u>EXEMPLES DE FORMULATION 1 (invention) et 2 (comparatif) :</u>**

On a préparé les compositions tinctoriales suivantes (teneurs en grammes) :

| Compositions | 1 | 2 (*) |
|---|---|---|
| 4-hydroxy 1-N-(β-hydroxyéthyl) indole | 0,886 | |
| 4-hydroxy 1-N-éthyl indole | | 0,805 |
| Para-aminophénol | 0,545 | 0,545 |
| Support de teinture commun | (**) | (**) |
| Eau déminéralisée q.s.p. | 100g | 100g |

(*) : exemple ne faisant pas partie de l'invention

(**) : support de teinture commun :

| | | |
|---|---|---|
| - Alcool oléique polyglycérolé à 2 moles de glycérol | 4,0 | g |
| - Alcool oléique polyglycérolé à 4 moles de glycérol à 78 % de matières actives (M.A.) | 5,69 | g M.A. |
| - Acide oléique | 3,0 | g |
| - Amine oléique à 2 moles d'oxyde d'éthylène vendue sous la dénomination commerciale ETHOMEEN O12 par la société AKZO | 7,0 | g |
| - Laurylamino succinamate de diéthylaminopropyle, sel de sodium à 55 % de M.A. | 3,0 | g M.A. |
| - Alcool oléique | 5,0 | g |
| - Diéthanolamide d'acide oléique | 12,0 | g |
| - Propylèneglycol | 3,5 | g |
| - Alcool éthylique | 7,0 | g |
| - Dipropylèneglycol | 0,5 | g |
| - Monométhyléther de propylèneglycol | 9,0 | g |
| - Métabisulfite de sodium à en solution aqueuse à 35 % de M.A. | 0,455 | g M.A. |
| - Acétate d'ammonium | 0,8 | g |
| - Antioxydant, séquestrant | q.s. | |
| - Parfum, conservateur | q.s. | |
| - Ammoniaque à 20 % de $NH_3$ | 10,0 | g |

Au moment de l'emploi, chaque composition tinctoriale 1 et 2 a été mélangée avec un poids égal d'eau oxygénée à 20 volumes (6% en poids).

Chaque mélange obtenu a été appliqué pendant 30 minutes, sur des mèches de cheveux gris naturels à 90 % de blancs. Après rinçage, lavage avec un shampooing standard et séchage, les mèches ont été teintes dans les nuances figurant dans le tableau I ci-dessous :

| **Exemple** | **1** | **2 (*)** |
|---|---|---|
| **Nuance obtenue** | Rouge cuivré soutenu | Rouge cuivré léger |

(*) : exemple ne faisant pas partie de l'invention

Les mèches ainsi teintes ont ensuite subi un test de résistance à la lumière (Xenotest).

Pour ce faire, les mèches de cheveux teintes ont été fixées sur un support (carton ou plastique). Ces supports ont été disposés sur des porte-échantillons que l'on a fait tourner autour d'une lampe Xénon pendant une durée de 40 heures sous un taux d'humidité relative de 25 ± 5 % et à une température de 42,5 ± 2,5°C.

La couleur des mèches a été évaluée dans le système MUNSELL, avant et après le test de résistance à la lumière, au moyen d'un colorimètre CM 2002 MINOLTA.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur de chaque mèche avant et après le test de résistance à la lumière reflète la dégradation de la coloration due à l'action de la lumière et a été calculée en appliquant la formule de NICKERSON : $\Delta E = 0{,}4\ Co\Delta H + 6\Delta V + 3\ \Delta C$, telle que décrite par exemple dans "Couleur, Industrie et Technique" ; pages 14-17 ; vol. n° 5 ; 1978.

Dans cette formule, **ΔE** représente la différence de couleur entre deux mèches, **ΔH**, **ΔV** et **ΔC** représentent la variation en valeur absolue des paramètres **H, V** et **C**, et **Co** représente la pureté de la mèche par rapport à laquelle on désire évaluer la différence de couleur (pureté de la mèche avant le test).

Les résultats sont donnés dans le tableau II ci-dessous :

| **EXEMPLE** | **Couleur avant le test** | **Couleur après le test** | **Dégradation de la coloration** | | | |
|---|---|---|---|---|---|---|
| | | | ΔH | ΔV | ΔC | **ΔE** |
| | | | | | | |
| **1** | 5,3 R 3,9 / 4,5 | 5,9 R 4,0 / 4,4 | 0,6 | 0,1 | 0,1 | **2,0** |
| **2 (*)** | 7,5 R 4,3 / 4,5 | 9,3 R 4,6 / 3,6 | 1,8 | 0,3 | 0,9 | **7,7** |

(*) : exemple ne faisant pas partie de l'invention

On constate que la coloration obtenue avec la composition tinctoriale de l'exemple 1 selon l'invention (renfermant du 4-hydroxy 1-N-(β-hydroxyéthyl) indole) résiste beaucoup mieux à l'action de la lumière que la coloration obtenue avec la composition tinctoriale de l'exemple 2 ne faisant pas partie de l'invention car contenant le 4-hydroxy 1-N-éthyl indole, composé ne répondant pas à la formule (I) définie précédemment, et correspondant à un composé de l'art antérieur tel que décrit dans l'EP-A-428 441.

## Revendications

**1.** Composition pour la teinture des fibres kératiniques et en particulier des fibres kératiniques humaines telles que les cheveux, caractérisée par le fait qu'elle comprend, dans un milieu approprié pour la teinture :

- à titre de coupleur, au moins un dérivé N-substitué du 4-hydroxy indole de formule (I), et/ou au moins l'un de ses sels d'addition avec un acide :

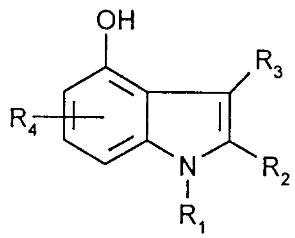

(I)

dans laquelle :

- $R_1$ représente un radical monohydroxyalkyle en $C_1$-$C_4$ ; polyhydroxyalkyle en $C_2$-$C_4$ ; alcoxy($C_1$-$C_4$)alkyle en $C_1$-$C_4$ ; hydroxyalcoxy($C_1$-$C_4$)alkyle en $C_1$-$C_4$ ; aminoalkyle en $C_1$-$C_4$ ; aminoalkyle en $C_1$-$C_4$ dont l'amine est mono ou disubstituée par un groupement alkyle en $C_1$-$C_4$, par un groupement acétyle, par un groupement monohydroxyalkyle en $C_1$-$C_4$ ou par un groupement polyhydroxyalkyle en $C_2$-$C_4$ ; alkyle($C_1$-$C_4$)thioalkyle en $C_1$-$C_4$, monohydroxyalkyl($C_1$-$C_4$)thioalkyle en $C_1$-$C_4$ ; polyhydroxyalkyl($C_2$-$C_4$)thioalkyle en $C_1$-$C_4$ ; carboxyalkyle en $C_1$-$C_4$ ; alcoxy($C_1$-$C_4$)carbonylalkyle en $C_1$-$C_4$ ; cyanoalkyle en $C_1$-$C_4$ ; trifluoroalkyle en $C_1$-$C_4$ ; halogénoalkyle en $C_1$-$C_4$ ; phosphoalkyle en $C_1$-$C_4$ ou sulfoalkyle en $C_1$-$C_4$ ;
- $R_2$ et $R_3$, identiques ou différents, représentent un atome d'hydrogène ou d'halogène ; un radical alkyle en $C_1$-$C_4$ ; carboxyle ; alcoxy($C_1$-$C_4$)carbonyle ou formyle ;
- $R_4$ représente un atome d'hydrogène ou d'halogène ; un radical alkyle en $C_1$-$C_4$ ; alcoxy en $C_1$-$C_4$ ; acétylamino ; monohydroxyalkyle en $C_1$-$C_5$ ; polyhydroxyalkyle en $C_2$-$C_4$ ; alcoxy($C_1$-$C_4$)alkyle en $C_1$-$C_4$ ; thiophène ; furane ; phényle ; phényle substitué par un atome d'halogène, un alkyle en $C_1$-$C_4$, un trifluorométhyle, un alcoxy en $C_1$-$C_4$, un amino ou par un amino mono- ou disubstitué par un radical alkyle en $C_1$-$C_4$ ; alkyl($C_1$-$C_4$)aminoalkyle en $C_1$-$C_4$ ou dialkyl($C_1$-$C_4$)aminoalkyle en $C_1$-$C_4$ ; les groupements alkyle, et alcoxy étant linéaires ou ramifiés et les atomes d'halogène pouvant être choisis parmi le chlore, le brome, l'iode et le fluor,
- et au moins une base d'oxydation.

2. Composition selon la revendication 1, caractérisée par le fait que les composés de formule (I) sont choisis parmi :

- le 4-hydroxy 1-N-(β-hydroxyéthyl) indole,
- le 4-hydroxy 1-N-(β-hydroxypropyl) indole,
- le 1-N-(β,γ-dihydroxypropyl) 4-hydroxy indole,
- le 4-hydroxy 1-N-(β-hydroxyéthyl) 5-méthyl indole,
- le 4-hydroxy 1-N-(β-hydroxypropyl) 5-méthyl indole,
- le 1-N-(β,γ-dihydroxypropyl) 4-hydroxy 5-méthyl indole,
- le 4-hydroxy 1-N-(β-hydroxyéthyl) 6-méthyl indole,
- le 4-hydroxy 1-N-(β-hydroxypropyl) 6-méthyl indole,
- le 1-N-(β,γ-dihydroxypropyl) 4-hydroxy 6-méthyl indole,
- le 5-benzyl 4-hydroxy 1-N-(β-hydroxyéthyl) indole,
- le 5-benzyl 4-hydroxy 1-N-(β-hydroxypropyl) indole,
- le 5-benzyl 1-N-(β,γ-dihydroxypropyl) 4-hydroxy indole,
- le 4-hydroxy 1-N-(β-hydroxyéthyl) 5-β-hydroxyéthyl indole,
- le 4-hydroxy 5-β-hydroxyéthyl 1-N-(β-hydroxypropyl) indole,
- le 1-N-(β,γ-dihydroxypropyl) 4-hydroxy 5-β-hydroxyéthyl indole,
- le 4-hydroxy 1-N-(β-hydroxyéthyl) 5-β,γ-dihydroxypropyl indole,
- le 4-hydroxy 1-N-(β-hydroxypropyl) 5-β,γ-dihydroxypropyl indole,
- le 1-N-(β,γ-dihydroxypropyl) 4-hydroxy 5-β,γ-dihydroxypropyl indole,
- le 1-N-(γ-diméthylaminopropyl) 4-hydroxy indole,
- le 1-N-éthylaminoéthyl 4-hydroxy indole

et leurs sels d'addition avec un acide.

3. Composition selon la revendication 2, caractérisée par le fait que les composés de formule (I) sont choisis parmi :

- le 4-hydroxy 1-N-(β-hydroxyéthyl) indole,
- le 4-hydroxy 1-N-(β-hydroxypropyl) indole,

- le 1-N-(β,γ-dihydroxypropyl) 4-hydroxy indole,
- le 4-hydroxy 1-N-(β-hydroxyéthyl) 5-méthyl indole,
- le 1-N-(γ-diméthylaminopropyl) 4-hydroxy indole,

et leur sels d'addition avec un acide.

**4.** Composition selon l'une quelconque des revendications précédentes, caractérisée par le fait que les sels d'addition avec un acide des composés de formule (I) sont choisis parmi les chlorhydrates, les bromhydrates, les sulfates et les tartrates.

**5.** Composition selon l'une quelconque des revendications précédentes caractérisée par le fait que le ou les composés de formule (I) représentent de 0,0005 à 12 % en poids du poids total de la composition tinctoriale.

**6.** Composition selon la revendication 5, caractérisée par le fait que le ou composés de formule (I) représentent de 0,005 à 6 % en poids du poids total de la composition tinctoriale.

**7.** Composition selon l'une quelconque des revendications précédentes caractérisée par le fait que la ou les bases d'oxydation sont choisies parmi les paraphénylènediamines, les bis-phénylalkylènediamines, les para-aminophénols, les ortho-aminophénols, les bases hétérocycliques, et leurs sels d'addition avec un acide.

**8.** Composition selon l'une quelconque des revendications précédentes, caractérisée par le fait que la ou les bases d'oxydation représentent de 0,0005 à 12 % en poids environ du poids total de la composition tinctoriale.

**9.** Composition selon la revendication 8, caractérisée par le fait que la ou les bases d'oxydation représentent de 0,005 à 6 % en poids environ du poids total de la composition tinctoriale.

**10.** Composition selon l'une quelconque des revendications précédentes, caractérisée par le fait qu'elle renferme en outre un ou plusieurs coupleurs additionnels différents des dérivés N-substitués du 4-hydroxyindole de formule (I) et/ou un ou plusieurs colorants directs.

**11.** Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**12.** Composition selon l'une quelconque des revendications précédentes, caractérisée par le fait qu'elle présente un pH compris entre 3 et 12.

**13.** Composition selon l'une quelconque des revendications précédentes, caractérisée par le fait qu'elle se présente sous forme de liquides, de crèmes, de gels, ou sous toute autre forme appropriée pour réaliser une teinture des fibres kératiniques, et notamment des cheveux humains.

**14.** Utilisation des dérivés N-substitués du 4-hydroxy indole de formule (I) telle que définie à l'une quelconque des revendications 1 à 4, à titre de coupleurs dans des compositions pour la teinture d'oxydation des fibres kératiniques et en particulier des fibres kératiniques humaines telles que les cheveux, en association avec au moins une base d'oxydation.

**15.** Procédé de teinture d'oxydation des fibres kératiniques et en particulier des fibres kératiniques humaines telles que les cheveux, caractérisé par le fait qu'on applique sur ces fibres au moins une composition tinctoriale telle que définie à l'une quelconque des revendications 1 à 13, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

**16.** Procédé selon la revendication 15, caractérisé par le fait que l'agent oxydant est choisi parmi le peroxyde d'hydrogène, le peroxyde d'urée, les bromates de métaux alcalins, les persels tels que les perborates et persulfates.

**17.** Dispositif à plusieurs compartiments, ou "kit" de teinture à plusieurs compartiments, dont un premier compartiment renferme une composition tinctoriale telle que définie à l'une quelconque des revendications 1 à 13 et un second

compartiment renferme une composition oxydante.

**18.** Dérivés N-substitués du 4-hydroxyindole de formule (I'), et leurs sels d'addition avec un acide :

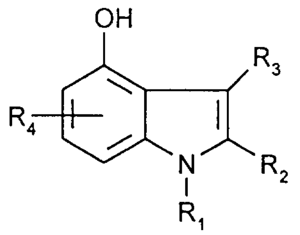

(I')

dans laquelle :

- $R_1$ représente un radical monohydroxyalkyle en $C_1$-$C_4$ ; polyhydroxyalkyle en $C_2$-$C_4$ ; alcoxy($C_1$-$C_4$)alkyle en $C_1$-$C_4$ ; hydroxyalcoxy($C_1$-$C_4$)alkyle en $C_1$-$C_4$ ; aminoalkyle en $C_1$-$C_4$ ; aminoalkyle en $C_1$-$C_4$ dont l'amine est mono ou disubstituée par un groupement alkyle en $C_1$-$C_4$, par un groupement acétyle, par un groupement monohydroxyalkyle en $C_1$-$C_4$ ou par un groupement polyhydroxyalkyle en $C_2$-$C_4$ ; alkyle($C_1$-$C_4$)thioalkyle en $C_1$-$C_4$, monohydroxyalkyl($C_1$-$C_4$)thioalkyle en $C_1$-$C_4$ ; polyhydroxyalkyl($C_2$-$C_4$)thioalkyle en $C_1$-$C_4$ ; carboxyalkyle en $C_1$-$C_4$ ; alcoxy($C_1$-$C_4$)carbonylalkyle en $C_1$-$C_4$ ; cyanoalkyle en $C_1$-$C_4$ ; trifluoroalkyle en $C_1$-$C_4$ ; halogénoalkyle en $C_1$-$C_4$ ; phosphoalkyle en $C_1$-$C_4$ ou sulfoalkyle en $C_1$-$C_4$ ;
- $R_2$ et $R_3$, identiques ou différents, représentent un atome d'hydrogène ou d'halogène ; un radical alkyle en $C_1$-$C_4$ ; carboxyle ; alcoxy($C_1$-$C_4$)carbonyle ou formyle ;
- $R_4$ représente un atome d'hydrogène ou d'halogène ; un radical alkyle en $C_1$-$C_4$ ; alcoxy en $C_1$-$C_4$ ; acétylamino ; monohydroxyalkyle en $C_1$-$C_5$ ; polyhydroxyalkyle en $C_2$-$C_4$ ; alcoxy($C_1$-$C_4$)alkyle en $C_1$-$C_4$ ; thiophène ; furane ; phényle ; phényle substitué par un atome d'halogéne, un alkyle en $C_1$-$C_4$, un trifluorométhyle, un alcoxy en $C_1$-$C_4$, un amino ou par un amino mono- ou disubstitué par un radical alkyle en $C_1$-$C_4$ ; alkyl($C_1$-$C_4$)aminoalkyle en $C_1$-$C_4$ ou dialkyl($C_1$-$C_4$)aminoalkyle en $C_1$-$C_4$ ;
  étant entendu que :
- lorsque $R_2$ et $R_4$ représentent simultanément un atome d'hydrogène, et que $R_1$ représente un groupement méthoxyméthyle ou γ-chloro-β-hydroxypropyle, alors $R_3$ ne peut représenter un radical formyle,
- et lorsque que $R_2$, $R_3$ et $R_4$ représentent simultanément un atome d'hydrogène, alors $R_1$ ne peut représenter un radical diméthylaminoéthyle.

**19.** Dérivés selon la revendication 18, caractérisé par le fait qu'ils sont choisis parmi :

- le 4-hydroxy 1-N-(β-hydroxyéthyl) indole,
- le 4-hydroxy 1-N-(β-hydroxypropyl) indole,
- le 1-N-(β,γ-dihydroxypropyl) 4-hydroxy indole,
- le 4-hydroxy 1-N-(β-hydroxyéthyl) 5-méthyl indole,
- le 4-hydroxy 1-N-(β-hydroxypropyl) 5-méthyl indole,
- le 1-N-(β,γ-dihydroxypropyl) 4-hydroxy 5-méthyl indole,
- le 4-hydroxy 1-N-(β-hydroxyéthyl) 6-méthyl indole,
- le 4-hydroxy 1-N-(β-hydroxypropyl) 6-méthyl indole,
- le 1-N-(β,γ-dihydroxypropyl) 4-hydroxy 6-méthyl indole,
- le 5-benzyl 4-hydroxy 1-N-(β-hydroxyéthyl) indole,
- le 5-benzyl 4-hydroxy 1-N-(β-hydroxypropyl) indole,
- le 5-benzyl 1-N-(β,γ-dihydroxypropyl) 4-hydroxy indole,
- le 4-hydroxy 1-N-(β-hydroxyéthyl) 5-β-hydroxyéthyl indole,
- le 4-hydroxy 5-β-hydroxyéthyl 1-N-(β-hydroxypropyl) indole,
- le 1-N-(β,γ-dihydroxypropyl) 4-hydroxy 5-β-hydroxyéthyl indole,
- le 4-hydroxy 1-N-(β-hydroxyéthyl) 5-β,γ-dihydroxypropyl indole,
- le 4-hydroxy 1-N-(β-hydroxypropyl) 5-β,γ-dihydroxypropyl indole,
- le 1-N-(β,γ-dihydroxypropyl) 4-hydroxy 5-β,γ-dihydroxypropyl indole,
- le 1-N-(γ-diméthylaminopropyl) 4-hydroxy indole,
- le 1-N-éthylaminoéthyl 4-hydroxy indole

et leurs sels d'addition avec un acide.

**20.** Dérivés selon la revendication 19, caractérisé par le fait qu'ils sont choisis parmi :

- le 4-hydroxy 1-N-(β-hydroxyéthyl) indole,
- le 4-hydroxy 1-N-(β-hydroxypropyl) indole,
- le 1-N-(β,γ-dihydroxypropyl) 4-hydroxy indole,
- le 4-hydroxy 1-N-(β-hydroxyéthyl) 5-méthyl indole,
- le 1-N-(γ-diméthylaminopropyl) 4-hydroxy indole,

et leurs sels d'addition avec un acide.

**21.** Procédé de préparation des dérivés N-substitués du 4-hydroxy indole de formule (I') telle que définie à la revendication 18, caractérisé par le fait qu'il consiste à faire réagir, dans une première étape, un 4-oxo 4,5,6,7-tetrahydro benzofurane avec une amine substituée, dans un milieu solvant dont la température est comprise entre 80 et 160°C, pour conduire à un dérivé 4-oxo 4,5,6,7-tetrahydroindolique, puis dans une deuxième étape, à aromatiser ledit dérivé 4-oxo 4,5,6,7-tetrahydroindolique par déshydrogénation catalytique en milieu solvant, à une température comprise entre 150 et 220°C, pour conduire à un dérivé N-substitué du 4-hydroxyindole de formule (I').

## Claims

**1.** Composition for dyeing keratinous fibres, and in particular human keratinous fibres such as hair, characterized in that it comprises, in a medium suitable for dyeing:

- as coupler, at least one N-substituted derivative of 4-hydroxyindole of formula (I), and/or at least one of its addition salts with an acid:

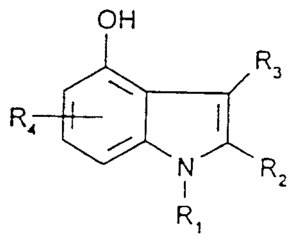

(I)

in which:

- $R_1$ represents a $C_1$-$C_4$ monohydroxyalkyl radical; a $C_2$-$C_4$ polyhydroxyalkyl radical; a $C_1$-$C_4$ alkoxy($C_1$-$C_4$)alkyl radical; a $C_1$-$C_4$ hydroxyalkoxy($C_1$-$C_4$)alkyl radical; a $C_1$-$C_4$ aminoalkyl radical; a $C_1$-$C_4$ aminoalkyl radical whose amine is mono- or disubstituted with a $C_1$-$C_4$ alkyl group, with an acetyl group, with a $C_1$-$C_4$ monohydroxyalkyl group or with a $C_2$-$C_4$ polyhydroxyalkyl group; a $C_1$-$C_4$ alkyl($C_1$-$C_4$)thioalkyl radical; a $C_1$-$C_4$ monohydroxyalkyl($C_1$-$C_4$)thioalkyl radical; a $C_2$-$C_4$ polyhydroxyalkyl($C_1$-$C_4$)thioalkyl radical; a $C_1$-$C_4$ carboxyalkyl radical; a $C_1$-$C_4$ alkoxy($C_1$-$C_4$)- carbonylalkyl radical; a $C_1$-$C_4$ cyanoalkyl radical; a $C_1$-$C_4$ trifluoroalkyl radical; a $C_1$-$C_4$ haloalkyl radical; a $C_1$-$C_4$ phosphoalkyl radical or a $C_1$-$C_4$ sulphoalkyl radical;
- $R_2$ and $R_3$, which are identical or different, represent a hydrogen or a halogen atom or a $C_1$-$C_4$ alkyl, a carboxyl, a $C_1$-$C_4$ alkoxycarbonyl or a formyl radical;
- $R_4$ represents a hydrogen or a halogen atom; a $C_1$-$C_4$ alkyl radical; a $C_1$-$C_4$ alkoxy radical; an acetylamino radical; a $C_1$-$C_5$ monohydroxyalkyl radical; a $C_2$-$C_4$ polyhydroxyalkyl radical; a $C_1$-$C_4$ alkoxy($C_1$-$C_4$)alkyl radical; a thiophene radical; a furan radical; a phenyl radical; a phenyl radical substituted with a halogen atom, a $C_1$-$C_4$ alkyl radical, a trifluoromethyl radical, a $C_1$-$C_4$ alkoxy radical, an amino radical or with an amino radical mono- or disubstituted with a $C_1$-$C_4$ alkyl radical; a $C_1$-$C_4$ alkyl($C_1$-$C_4$)aminoalkyl radical or a $C_1$-$C_4$ dialkyl($C_1$-$C_4$)aminoalkyl radical; the alkyl and alkoxy groups being linear or branched and it being possible for the halogen atoms to be chosen from chlorine, bromine, iodine and fluorine;
- and at least one oxidation base.

**2.** Composition according to Claim 1, characterized in that the compounds of formula (I) are chosen from:

- 4-hydroxy-1-N-(β-hydroxyethyl)indole,

- 4-hydroxy-1-N-(β-hydroxypropyl)indole,
- 1-N-(β,γ-dihydroxypropyl)-4-hydroxyindole,
- 4-hydroxy-1-N-(β-hydroxyethyl)-5-methylindole,
- 4-hydroxy-1-N-(β-hydroxypropyl)-5-methylindole,
- 1-N-(β,γ-dihydroxypropyl)-4-hydroxy-5-methylindole,
- 4-hydroxy-1-N-(β-hydroxyethyl)-6-methylindole,
- 4-hydroxy-1-N-(β-hydroxypropyl)-6-methylindole,
- 1-N-(β,γ-dihydroxypropyl)-4-hydroxy-6-methylindole,
- 5-benzyl-4-hydroxy-1-N-(β-hydroxyethyl)indole,
- 5-benzyl-4-hydroxy-1-N-(β-hydroxypropyl)indole,
- 5-benzyl-1-N-(β,γ-dihydroxypropyl)-4-hydroxyindole,
- 4-hydroxy-1-N-(β-hydroxyethyl)-5-β-hydroxy-ethylindole,
- 4-hydroxy-5-β-hydroxyethyl-1-N-(β-hydroxypropyl)indole,
- 1-N-(β,γ-dihydroxypropyl)-4-hydroxy-5-β-hydroxyethylindole,
- 4-hydroxy-1-N-(β-hydroxyethyl)-5-β,γ-dihydroxypropylindole,
- 4-hydroxy-1-N-(β-hydroxypropyl)-5-β,γ-dihydroxypropylindole,
- 1-N-(β,γ-dihydroxypropyl)-4-hydroxy-5-β,γ-dihydroxypropylindole,
- 1-N-(γ-dimethylaminopropyl)-4-hydroxyindole,
- 1-N-ethylaminoethyl-4-hydroxyindole

and their addition salts with an acid.

**3.** Composition according to Claim 2, characterized in that the compounds of formula (I) are chosen from:

- 4-hydroxy-1-N-(β-hydroxyethyl)indole,
- 4-hydroxy-1-N-(β-hydroxypropyl)indole,
- 1-N-(β,γ-dihydroxypropyl)-4-hydroxyindole,
- 4-hydroxy-1-N-(β-hydroxyethyl)-5-methylindole,
- 1-N-(γ-dimethylaminopropyl)-4-hydroxyindole

and their addition salts with an acid.

**4.** Composition according to any one of the preceding claims, characterized in that the addition salts with an acid of the compounds of formula (I) are chosen from the hydrochlorides, hydrobromides, sulphates and tartrates.

**5.** Composition according to any one of the preceding claims, characterized in that the compound(s) of formula (I) represent from 0.0005 to 12 % by weight of the total weight of the dyeing composition.

**6.** Composition according to Claim 5, characterized in that the compound(s) of formula (I) represent from 0.005 to 6 % by weight of the total weight of the dyeing composition.

**7.** Composition according to any one of the preceding claims, characterized in that the oxidation base(s) are chosen from para-phenylenediamines, bis-phenylalkylenediamines, para-aminophenols, ortho-aminophenols, heterocyclic bases, and their addition salts with an acid.

**8.** Composition according to any one of the preceding claims, characterized in that the oxidation base(s) represent from 0.0005 to 12 % by weight approximately of the total weight of the dyeing composition.

**9.** Composition according to Claim 8, characterized in that the oxidation base(s) represent from 0.005 to 6 % by weight approximately of the total weight of the dyeing composition.

**10.** Composition according to any one of the preceding claims, characterized in that it contains, in addition, one or more additional couplers different from the N-substituted derivatives of 4-hydroxyindole of formula (I) and/or one or more direct dyes.

**11.** Composition according to any one of the preceding claims, characterized in that the medium suitable for dyeing (or vehicle) consists of water or of a mixture of water and at least one organic solvent chosen from $C_1$-$C_4$ lower alkanols, glycerol, glycols and glycol ethers, aromatic alcohols, similar products and mixtures thereof.

**12.** Composition according to any one of the preceding claims, characterized in that it has a pH of between 3 and 12.

**13.** Composition according to any one of the preceding claims, characterized in that it is provided in the form of liquids, creams, gels, or in any other form suitable for dyeing keratinous fibres, and especially human hair.

**14.** Use of the N-substituted derivatives of 4-hydroxyindole of formula (I) as defined in any one of Claims 1 to 4, as couplers in compositions for the oxidation dyeing of keratinous fibres, and in particular human keratinous fibres such as hair, in combination with at least one oxidation base.

**15.** Process for the oxidation dyeing of keratinous fibres, and in particular human keratinous fibres such as hair, characterized in that at least one dyeing composition as defined in any one of Claims 1 to 13 is applied to these fibres, the colour being developed at acid, neutral or alkaline pH using an oxidizing agent which is added to the dyeing composition just at the time of use or which is present in an oxidizing composition applied simultaneously or sequentially in a separate stage.

**16.** Process according to Claim 15, characterized in that the oxidizing agent is chosen from hydrogen peroxide, urea peroxide, alkali metal bromates and persalts such as perborates and persulphates.

**17.** Multi-compartment device or multi-compartment dyeing "kit" in which a first compartment contains a dyeing composition as defined in any one of Claims 1 to 13 and a second compartment contains an oxidizing composition.

**18.** N-substituted derivatives of 4-hydroxyindole of formula (I'), and their addition salts with an acid:

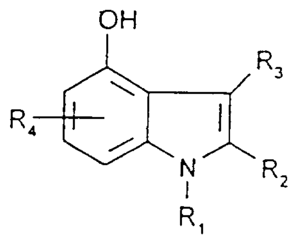

(I')

in which:

- $R_1$ represents a $C_1$-$C_4$ monohydroxyalkyl radical; a $C_2$-$C_4$ polyhydroxyalkyl radical; a $C_1$-$C_4$ alkoxy-($C_1$-$C_4$)alkyl radical; a $C_1$-$C_4$ hydroxyalkoxy($C_1$-$C_4$)alkyl radical; a $C_1$-$C_4$ aminoalkyl radical; a $C_1$-$C_4$ aminoalkyl radical whose amine is mono- or disubstituted with a $C_1$-$C_4$ alkyl group, with an acetyl group, with a $C_1$-$C_4$ monohydroxyalkyl group or with a $C_2$-$C_4$ polyhydroxyalkyl group; a $C_1$-$C_4$ alkyl($C_1$-$C_4$)thioalkyl radical; a $C_1$-$C_4$ monohydroxyalkyl($C_1$-$C_4$)thioalkyl radical; a $C_2$-$C_4$ polyhydroxyalkyl($C_1$-$C_4$)thioalkyl radical; a $C_1$-$C_4$ carboxyalkyl radical; a $C_1$-$C_4$ alkoxy($C_1$-$C_4$)- carbonylalkyl radical; a $C_1$-$C_4$ cyanoalkyl radical; a $C_1$-$C_4$ trifluoroalkyl radical; a $C_1$-$C_4$ haloalkyl radical; a $C_1$-$C_4$ phosphoalkyl radical or a $C_1$-$C_4$ sulphoalkyl radical;
- $R_2$ and $R_3$, which are identical or different, represent a hydrogen or a halogen atom or a $C_1$-$C_4$ alkyl, a carboxyl, an alkoxy($C_1$-$C_4$)carbonyl or a formyl radical;
- $R_4$ represents a hydrogen or a halogen atom; a $C_1$-$C_4$ alkyl radical; a $C_1$-$C_4$ alkoxy radical; an acetylamino radical; a $C_1$-$C_5$ monohydroxyalkyl radical; a $C_2$-$C_4$ polyhydroxyalkyl radical; a $C_1$-$C_4$ alkoxy($C_1$-$C_4$)alkyl radical; a thiophene radical; a furan radical; a phenyl radical; a phenyl radical substituted with a halogen atom, a $C_1$-$C_4$ alkyl radical, a trifluoromethyl radical, a $C_1$-$C_4$ alkoxy radical, an amino radical or with an amino radical mono- or disubstituted with a $C_1$-$C_4$ alkyl radical; a $C_1$-$C_4$ alkyl($C_1$-$C_4$)aminoalkyl radical or a $C_1$-$C_4$ dialkyl($C_1$-$C_4$)aminoalkyl radical;

  it being understood that:
- when $R_2$ and $R_4$ simultaneously represent a hydrogen atom, and $R_1$ represents a methoxymethyl or $\gamma$-chloro-$\beta$-hydroxypropyl group, then $R_3$ cannot represent a formyl radical,
- and when $R_2$, $R_3$ and $R_4$ simultaneously represent a hydrogen atom, then $R_1$ cannot represent a dimethylaminoethyl radical.

**19.** Derivatives according to Claim 18, characterized in that they are chosen from:

- 4-hydroxy-1-N-(β-hydroxyethyl)indole,
- 4-hydroxy-1-N-(β-hydroxypropyl)indole,
- 1-N-(β,γ-dihydroxypropyl)-4-hydroxyindole,
- 4-hydroxy-1-N-(β-hydroxyethyl)-5-methylindole,
- 4-hydroxy-1-N-(β-hydroxypropyl)-5-methylindole,
- 1-N-(β,γ-dihydroxypropyl)-4-hydroxy-5-methylindole,
- 4-hydroxy-1-N-(β-hydroxyethyl)-6-methylindole,
- 4-hydroxy-1-N-(β-hydroxypropyl)-6-methylindole,
- 1-N-(β,γ-dihydroxypropyl)-4-hydroxy-6-methylindole,
- 5-benzyl-4-hydroxy-1-N-(β-hydroxyethyl)indole,
- 5-benzyl-4-hydroxy-1-N-(β-hydroxypropyl)indole,
- 5-benzyl-1-N-(β,γ-dihydroxypropyl)-4-hydroxyindole,
- 4-hydroxy-1-N-(β-hydroxyethyl)-5-β-hydroxyethylindole,
- 4-hydroxy-5-β-hydroxyethyl-1-N-(β-hydroxypropyl)indole,
- 1-N-(β,γ-dihydroxypropyl)-4-hydroxy-5-β-hydroxyethylindole,
- 4-hydroxy-1-N-(β-hydroxyethyl)-5-β,γ-dihydroxypropylindole,
- 4-hydroxy-1-N-(β-hydroxypropyl)-5-β,γ-dihydroxypropylindole,
- 1-N-(β,γ-dihydroxypropyl)-4-hydroxy-5-β,y-dihydroxypropylindole,
- 1-N-(γ-dimethylaminopropyl)-4-hydroxyindole,
- 1-N-ethylaminoethyl-4-hydroxyindole

and their addition salts with an acid.

**20.** Derivatives according to Claim 19, characterized in that they are chosen from:

- 4-hydroxy-1-N-(β-hydroxyethyl)indole,
- 4-hydroxy-1-N-(β-hydroxypropyl)indole,
- 1-N-(β,γ-dihydroxypropyl)-4-hydroxyindole,
- 4-hydroxy-1-N-(β-hydroxyethyl)-5-methylindole,
- 1-N-(γ-dimethylaminopropyl)-4-hydroxyindole

and their addition salts with an acid.

**21.** Process for the preparation of the N-substituted derivatives of 4-hydroxyindole of formula (I') as defined in Claim 18, characterized in that it consists in reacting, in a first stage, a 4-oxo-4,5,6,7-tetrahydrobenzofuran with a substituted amine, in a solvent medium whose temperature is between 80 and 160°C, to give a 4-oxo-4,5,6,7-tetrahydroindole derivative, and then in a second stage, in aromatizing the said 4-oxo-4,5,6,7-tetrahydroindole derivative by catalytic dehydrogenation in a solvent medium, at a temperature of between 150 and 220°C, to give an N-substituted derivative of 4-hydroxyindole of formula (I').

## Patentansprüche

**1.** Zusammensetzung zum Färben von Keratinfasern und insbesondere menschlichen Keratinfasern, wie dem Haar, dadurch gekennzeichnet, daß
sie in einem zum Färben geeigneten Medium enthält:

- als Kuppler mindestens ein N-substituiertes 4-Hydroxyindolderivat der Formel (I) und/oder mindestens eines der Additionssalze dieser Verbindungen mit einer Säure:

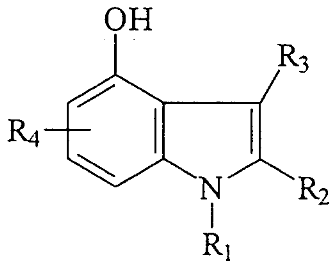

(I)

worin bedeuten:

$R_1$ $C_{1-4}$-Monohydroxyalkyl, $C_{2-4}$-Polyhydroxyalkyl, $C_{1-4}$-Alkoxy-$C_{1-4}$-alkyl, $C_{1-4}$-Hydroxyalkoxy- $C_{1-4}$-alkyl, $C_{1-4}$-Aminoalkyl, $C_{1-4}$-Aminoalkyl, wobei die Aminogruppe ein- oder zweifach mit einer $C_{1-4}$-Alkylgruppe, Acetylgruppe, $C_{1-4}$-Monohydroxyalkylgruppe oder $C_{2-4}$-Polyhydroxyalkylgruppe substituiert ist, $C_{1-4}$-Alkyl-$C_{1-4}$-thioalkyl, $C_{1-4}$-Monohydroxyalkyl-$C_{1-4}$-thioalkyl, $C_{2-4}$-Polyhydroxyalkyl-$C_{1-4}$-thioalkyl, $C_{1-4}$-Carboxyalkyl, $C_{1-4}$-Alkoxy-$C_{1-4}$-carbonylalkyl, $C_{1-4}$-Cyanoalkyl, $C_{1-4}$-Trifluoralkyl, $C_{1-4}$-Halogenalkyl, $C_{1-4}$-Phosphoalkyl oder $C_{1-4}$-Sulfoalkyl;

$R_2$ und $R_3$, die identisch oder voneinander verschieden sind, Wasserstoff, Halogen, $C_{1-4}$-Alkyl, Carboxy, $C_{1-4}$-Alkoxycarbonyl oder Formyl;

$R_4$ Wasserstoff, Halogen, $C_{1-4}$-Alkyl, $C_{1-4}$-Alkoxy, Acetylamino, $C_{1-5}$-Monohydroxyalkyl, $C_{2-4}$-Polyhydroxyalkyl, $C_{1-4}$-Alkoxy-$C_{1-4}$-alkyl, Thiophen, Furan, Phenyl, Phenyl, das mit Halogen, $C_{1-4}$-Alkyl, Trifluormethyl, $C_{1-4}$-Alkoxy, Amino oder Amino, das ein- oder zweifach mit einer $C_{1-4}$-Alkylgruppe substituiert ist, substituiert ist, $C_{1-4}$-Alkyl-$C_{1-4}$-aminoalkyl oder $C_{1-4}$-Dialkyl-$C_{1-4}$-aminoalkyl, wobei die Alkyl- und Alkoxygruppen geradkettig oder verzweigt vorliegen können und die Halogenatome unter Chlor, Brom, Iod und Fluor ausgewählt sein können.

und

- mindestens eine Oxidationsbase.

2. Zusammensetzung nach Anspruch 1, dadurch gekennzeichnet, daß die Verbindungen der Formel (I) ausgewählt sind unter:

4-Hydroxy-1-N-(β-hydroxyethyl)-indol,
4-Hydroxy-1-N-(β-hydroxypropyl)-indol,
1-N-(β,γ-Dihydroxypropyl)-4-hydroxy-indol,
4-Hydroxy-1-N-(β-hydroxyethyl)-5-methyl-indol,
4-Hydroxy-1-N-(β-hydroxypropyl)-5-methyl-indol,
1-N-(β,γ-Dihydroxypropyl)-4-hydroxy-5-methyl-indol,
4-Hydroxy-1-N-(β-hydroxyethyl)-6-methyl-indol,
4-Hydroxy-1-N-(β-hydroxypropyl)-6-methyl-indol,
1-N-(β,γ-Dihydroxypropyl)-4-hydroxy-6-methyl-indol,
5-Benzyl-4-hydroxy-1-N-(β-hydroxyethyl)-indol,
5-Benzyl-4-hydroxy-1-N-(β-hydroxypropyl)-indol,
5-Benzyl-1-N-(β,γ-dihydroxypropyl)-4-hydroxy-indol,
4-Hydroxy-1-N-(β-hydroxyethyl)-5-β-hydroxyethyl-indol,
4-Hydroxy-5-β-hydroxyethyl -1-N-(β-hydroxypropyl)-indol,
1-N-(β,γ-Dihydroxypropyl)-4-hydroxy-5-β-hydroxyethyl-indol,
4-Hydroxy-1-N-(β-hydroxyethyl)-5-β,γ-dihydroxypropyl-indol,

4-Hydroxy-1-N-(β-hydroxypropyl)-5-β,γ-dihydroxypropyl-indol,
1-N-(β,γ-Dihydroxypropyl)-4-hydroxy-5-β,γ-dihydroxypropyl-indol,
1-N-(γ-Dimethylaminopropyl)-4-hydroxy-indol,
1-N-Ethylaminoethyl-4-hydroxy-indol, und

den Additionssalzen dieser Verbindungen mit einer Säure.

**3.** Zusammensetzung nach Anspruch 3, dadurch gekennzeichnet, daß die Verbindungen der Formel (I) ausgewählt sind unter:

4-Hydroxy-1-N-(β-hydroxyethyl)-indol,
4-Hydroxy-1-N-(β-hydroxypropyl)-indol,
1-N-(β,γ-Dihydroxypropyl)-4-hydroxy-indol,
4-Hydroxy-1-N-(β-hydroxyethyl)-5-methyl-indol,
1-N-(γ-Dimethylaminopropyl)-4-hydroxy-indol und den Additionssalzen dieser Verbindungen mit einer Säure.

**4.** Zusammensetzung nach einem der vorhergehenden Ansprüche, dadurch gekennzeichnet, daß die Additionssalze der Verbindungen der Formel (I) mit einer Säure unter den Hydrochloriden, Hydrobromiden, Sulfaten und Tartraten ausgewählt sind.

**5.** Zusammensetzung nach einem der vorhergehenden Ansprüche, dadurch gekennzeichnet, daß die Verbindung oder die Verbindungen der Formel (I) 0,0005 bis 12 Gew.-% des Gesamtgewichts der Färbemittelzusammensetzung ausmachen.

**6.** Zusammensetzung nach Anspruch 5, dadurch gekennzeichnet, daß die Verbindung oder die Verbindungen der Formel (I) 0,005 bis 6 Gew.-% des Gesamtgewichts der Färbemittelzusammensetzung ausmachen.

**7.** Zusammensetzung nach einem der vorhergehenden Ansprüche, dadurch gekennzeichnet, daß die Oxidationsbase oder die Oxidationsbasen unter den p-Phenylendiaminen, Bisphenylalkylendiaminen, p-Aminophenolen, o-Aminophenolen, heterocyclischen Basen und den Additionssalzen dieser Verbindungen mit einer Säure ausgewählt sind.

**8.** Zusammensetzung nach einem der vorhergehenden Ansprüche, dadurch gekennzeichnet, daß die Oxidationsbase oder die Oxidationsbasen etwa 0,0005 bis 12 Gew.-% des Gesamtgewichts der Färbemittelzusammensetzung ausmachen.

**9.** Zusammensetzung nach Anspruch 8, dadurch gekennzeichnet, daß die Oxidationsbase oder die Oxidationsbasen etwa 0,005 bis 6 Gew.-% des Gesamtgewichts der Färbemittelzusammensetzung ausmachen.

**10.** Zusammensetzung nach einem der vorhergehenden Ansprüche, dadurch gekennzeichnet, daß sie ferner einen oder mehrere zusätzliche Kuppler, die von den N-substituierten 4-Hydroxyindolderivaten der Formel (I) verschieden sind, und/oder einen oder mehrere Direktfarbstoffe enthält.

**11.** Zusammensetzung nach einem der vorhergehenden Ansprüche, dadurch gekennzeichnet, daß das zum Färben geeignete Medium (oder der Träger) aus Wasser oder einem Gemisch aus Wasser und mindestens einem organischen Lösungsmittel besteht, das unter den niederen $C_{1-4}$-Alkanolen, Glycerin, den Glykolen und Glykolethern, den aromatischen Alkoholen, analogen Produkten und deren Gemischen ausgewählt ist.

**12.** Zusammensetzung nach einem der vorhergehenden Ansprüche, dadurch gekennzeichnet, daß sie einen pH-Wert im Bereich von 3 bis 12 aufweist.

**13.** Zusammensetzung nach einem der vorhergehenden Ansprüche, dadurch gekennzeichnet, daß sie in Form von Flüssigkeiten, Cremes, Gelen oder beliebigen weiteren Formen vorliegt, die dazu geeignet sind, eine Färbung von Keratinfasern und insbesondere menschlichen Keratinfasern durchzuführen.

**14.** Verwendung von N-substituierten 4-Hydroxyindolderivaten der Formel (I) nach einem der Ansprüche 1 bis 4 als Kuppler in Kombination mit mindestens einer Oxidationsbase zum Färben von Keratinfasern und insbesondere menschlichen Keratinfasern, wie dem Haar.

**15.** Verfahren zum oxidativen Färben von Keratinfasern und insbesondere menschlichen Keratinfasern, wie dem Haar, dadurch gekennzeichnet, daß auf die Fasern mindestens eine Färbemittelzusammensetzung nach einem der Ansprüche 1 bis 13 aufgetragen und die Farbe bei einem sauren, neutralen oder alkalischen pH-Wert mit einem Oxidationsmittel entwickelt wird, das bei der Anwendung zu dieser Färbemittelzusammensetzung gegeben wird oder das in einer oxidierenden Zusammensetzung vorliegt, die gleichzeitig oder getrennt davon anschließend aufgetragen wird.

**16.** Verfahren nach Anspruch 15, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

**17.** Vorrichtung mit mehreren Abteilungen oder "Kit" zum Färben mit mehreren Abteilungen, wobei eine erste Abteilung eine Färbemittelzusammensetzung nach einem der Ansprüche 1 bis 13 und eine zweite Abteilung eine oxidierende Zusammensetzung enthält.

**18.** N-substituierte 4-Hydroxyindolderivate der Formel (I') und die Additionssalze dieser Verbindungen mit einer Säure:

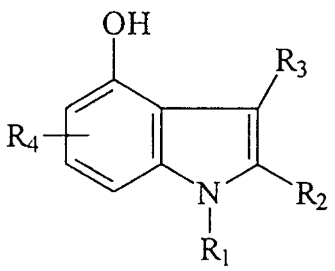

(I')

worin bedeuten:

$R_1$ $C_{1-4}$-Monohydroxyalkyl, $C_{2-4}$-Polyhydroxyalkyl, $C_{1-4}$-Alkoxy-$C_{1-4}$-alkyl, $C_{1-4}$-Hydroxyalkoxy-$C_{1-4}$-alkyl, $C_{1-4}$-Aminoalkyl, $C_{1-4}$-Aminoalkyl, wobei die Aminogruppe ein- oder zweifach mit einer $C_{1-4}$-Alkylgruppe, Acetylgruppe, $C_{1-4}$-Monohydroxyalkylgruppe oder $C_{2-4}$-Polyhydroxyalkylgruppe substituiert ist, $C_{1-4}$-Alkyl-$C_{1-4}$-thioalkyl, $C_{1-4}$-Monohydroxyalkyl-$C_{1-4}$-thioalkyl, $C_{2-4}$-Polyhydroxyalkyl-$C_{1-4}$-thioalkyl, $C_{1-4}$-Carboxyalkyl, $C_{1-4}$-Alkoxy-$C_{1-4}$-carbonylalkyl, $C_{1-4}$-Cyanoalkyl, $C_{1-4}$-Trifluoralkyl, $C_{1-4}$-Halogenalkyl, $C_{1-4}$-Phosphoalkyl oder $C_{1-4}$-Sulfoalkyl;

$R_2$ und $R_3$, die identisch oder voneinander verschieden sind, Wasserstoff, Halogen, $C_{1-4}$-Alkyl, Carboxy, $C_{1-4}$-Alkoxycarbonyl oder Formyl;

$R_4$ Wasserstoff, Halogen, $C_{1-4}$-Alkyl, $C_{1-4}$-Alkoxy, Acetylamino, $C_{1-5}$-Monohydroxyalkyl, $C_{2-4}$-Polyhydroxyalkyl, $C_{1-4}$-Alkoxy-$C_{1-4}$-alkyl, Thiophen, Furan, Phenyl, Phenyl, das mit Halogen, $C_{1-4}$-Alkyl, Trifluormethyl, $C_{1-4}$-Alkoxy, Amino oder Amino, das ein- oder zweifach mit einer $C_{1-4}$-Alkylgruppe substituiert ist, substituiert ist, $C_{1-4}$-Alkyl-$C_{1-4}$-aminoalkyl oder $C_{1-4}$-Dialkyl-$C_{1-4}$-aminoalkyl;
mit der Maßgabe, daß

$R_3$ keine Formylgruppe bedeuten kann, wenn $R_2$ und $R_4$ gleichzeitig Wasserstoff bedeuten und $R_1$ Methoxymethyl oder γ-Chlor-β-hydroxypropyl bedeutet, und

$R_1$ keine Dimethylaminoethylgruppe bedeuten kann, wenn $R_2$, $R_3$ und $R_4$ gleichzeitig Wasserstoff bedeuten.

**19.** Derivate nach Anspruch 18, dadurch gekennzeichnet, daß sie ausgewählt sind unter:

4-Hydroxy-1-N-(β-hydroxyethyl)-indol,
4-Hydroxy-1-N-(β-hydroxypropyl)-indol,
1-N-(β,γ-Dihydroxypropyl)-4-hydroxy-indol,
4-Hydroxy-1-N-(β-hydroxyethyl)-5-methyl-indol,

4-Hydroxy-1-N-(β-hydroxypropyl)-5-methyl-indol,
1-N-(β,γ-Dihydroxypropyl)-4-hydroxy-5-methyl-indol,
4-Hydroxy-1-N-(β-hydroxyethyl)-6-methyl-indol,
4-Hydroxy-1-N-(β-hydroxypropyl)-6-methyl-indol,
1-N-(β,γ-Dihydroxypropyl)-4-hydroxy-6-methyl-indol,
5-Benzyl-4-hydroxy-1-N-(β-hydroxyethyl)-indol,
5-Benzyl-4-hydroxy-1-N-(β-hydroxypropyl)-indol,
5-Benzyl-1-N-(β,γ-dihydroxypropyl)-4-hydroxy-indol,
4-Hydroxy-1-N-(β-hydroxyethyl)-5-β-hydroxyethyl-indol,
4-Hydroxy-5-β-hydroxyethyl -1-N-(β-hydroxypropyl)-indol,
1-N-(β,γ-Dihydroxypropyl)-4-hydroxy-5-β-hydroxyethyl-indol,
4-Hydroxy-1-N-(β-hydroxyethyl)-5-β,γ-dihydroxypropyl-indol,
4-Hydroxy-1-N-(β-hydroxypropyl)-5-β,γ-dihydroxypropyl-indol,
1-N-(β,γ-Dihydroxypropyl)-4-hydroxy-5-β,γ-dihydroxypropyl-indol,
1-N-(γ-Dimethylaminopropyl)-4-hydroxy-indol,
1-N-Ethylaminoethyl-4-hydroxy-indol, und
den Additionssalzen dieser Verbindungen mit einer Säure.

**20.** Derivate nach Anspruch 19, dadurch gekennzeichnet, daß sie ausgewählt sind unter:

4-Hydroxy-1-N-(β-hydroxyethyl)-indol,
4-Hydroxy-1-N-(β-hydroxypropyl)-indol,
1-N-(β,γ-Dihydroxypropyl)-4-hydroxy-indol,
4-Hydroxy-1-N-(β-hydroxyethyl)-5-methyl-indol,
1-N-(γ-Dimethylaminopropyl)-4-hydroxy-indol und
den Additionssalzen dieser Verbindungen mit einer Säure.

**21.** Verfahren zur Herstellung von N-substituierten 4-Hydroxyindolderivaten der Formel (I') nach Anspruch 18, dadurch gekennzeichnet, daß in einem ersten Schritt ein 4-Oxo-4,5,6,7-Tetrahydro-benzofuran mit einem substituierten Amin in einem Lösungsmittelmedium einer Temperatur von 80 bis 160 °C umgesetzt wird, wodurch ein 4-Oxo-4,5,6,7-Tetrahydroindolderivat hergestellt wird, worauf in einem zweiten Schritt das 4-Oxo-4,5,6,7-Tetrahydroindolderivat durch katalytische Dehydrierung in einem Lösungsmittelmedium bei einer Temperatur im Bereich von 150 bis 220 °C aromatisiert wird, wodurch ein N-substituiertes 4-Hydroxyindolderivat der Formel (I') hergestellt wird.